# EUROPÄISCHE PATENTANMELDUNG

(11) **EP 4 830 661 A1**
(43) Veröffentlichungstag der Anmeldung: **28.10.2026**
(21) Anmeldenummer: 26163561.9
(22) Anmeldetag: 10.03.2026
(51) Int. Cl.: A61B 90/70, A61L 2/00, A61L 2/18, A61L 2/24, A61L 2/26

(54) **REINIGUNGSGERÄT FÜR MEDIZINISCHE INSTRUMENTE UND VERFAHREN ZUM BETREIBEN EINES REINIGUNGSGERÄTS**

(30) Priorität: 23.04.2025 LU 601279
(71) Anmelder: Miele & Cie. KG, 33332 Gütersloh (DE)
(72) Erfinder: Goldberg, Gregor, 48231 Warendorf (DE)

(57) **Zusammenfassung**

Ein Reinigungsgerät (WD) für medizinische Instrumente weist einen Reinigungsraum (SK), eine Pflegemittelleitung (105), eine Abschirmeinrichtung (200) und eine Abschirmmediumsleitung (205) auf. Der Reinigungsraum (SK) weist einen ersten Reinigungsbereich auf, in dem eine Aufnahmeeinrichtung (206) zum Aufnehmen zumindest eines der medizinischen Instrumente angeordnet ist. Die Pflegemittelleitung (105) ist ausgebildet, um ein Pflegemittel an eine Schnittstelle (KS2) zu der Aufnahmeeinrichtung (206) zu leiten, um das in der Aufnahmeeinrichtung (206) aufgenommene medizinische Instrument während eines Pflegebetriebs mit dem Pflegemittel zu pflegen. Die Abschirmeinrichtung (200) ist innerhalb des Reinigungsraums (SK) angeordnet und ist ausgebildet, um ein Abschirmmedium auszugeben, um den ersten Reinigungsbereich von einem sich von dem ersten Reinigungsbereich unterscheidenden zweiten Reinigungsbereich des Reinigungsraums (SK) zu separieren, um während des Pflegebetriebs ein Übertragen des Pflegemittels von dem ersten Reinigungsbereich in den zweiten Reinigungsbereich zu verhindern. Die Abschirmmediumsleitung (205) ist ausgebildet, um das Abschirmmedium an eine Koppelstelle (KS3) zu der Abschirmeinrichtung (200) zu leiten.

## Beschreibung

Die Erfindung betrifft ein Reinigungsgerät für medizinische Instrumente und ein Verfahren zum Betreiben eines Reinigungsgeräts.

Der hier vorgestellte Ansatz stellt sich die Aufgabe, ein verbessertes Reinigungsgerät für medizinische Instrumente sowie ein verbessertes Verfahren zum Betreiben eines Reinigungsgeräts zu schaffen.

Erfindungsgemäß wird diese Aufgabe durch ein Reinigungsgerät für medizinische Instrumente und ein Verfahren zum Betreiben eines Reinigungsgeräts mit den Merkmalen bzw. Schritten der Hauptansprüche gelöst. Vorteilhafte Ausgestaltungen und Weiterbildungen der Erfindung ergeben sich aus den nachfolgenden Unteransprüchen.

Die mit der Erfindung erreichbaren Vorteile bestehen darin, dass während eines Pflegebetriebs in einem Reinigungsgerät medizinische Instrumente mit einem Pflegemittel gepflegt werden können, während andere medizinische Instrumente, welche nicht mit dem Pflegemittel gepflegt werden sollen, nicht von dem Pflegemittel kontaktiert werden.

Ein Reinigungsgerät für medizinische Instrumente weist einen Reinigungsraum, eine Pflegemittelleitung, eine Abschirmeinrichtung und eine Abschirmmediumsleitung auf. Der Reinigungsraum weist einen ersten Reinigungsbereich auf, in dem eine Aufnahmeeinrichtung zum Aufnehmen zumindest eines der medizinischen Instrumente angeordnet oder anordenbar ist. Die Pflegemittelleitung ist dazu ausgebildet, um ein Pflegemittel an eine Schnittstelle zu der Aufnahmeeinrichtung zu leiten, um das in oder an der Aufnahmeeinrichtung aufgenommene medizinische Instrument während eines Pflegebetriebs mit dem Pflegemittel zu pflegen. Die Abschirmeinrichtung ist innerhalb des Reinigungsraums angeordnet oder anordenbar und ist ausgebildet, um ein Abschirmmedium auszugeben, um den ersten Reinigungsbereich von einem sich von dem ersten Reinigungsbereich unterscheidenden zweiten Reinigungsbereich des Reinigungsraums zu separieren, um während des Pflegebetriebs ein Übertragen des Pflegemittels von dem ersten Reinigungsbereich in den zweiten Reinigungsbereich zu verhindern. Die Abschirmmediumsleitung ist ausgebildet, um das Abschirmmedium an eine Koppelstelle zu der Abschirmeinrichtung zu leiten.

Einige medizinische Instrumente erfordern, beispielsweise nach einem Reinigungsbetrieb, in welchem die medizinischen Instrumente gereinigt werden, eine Pflege mit einem Pflegemittel.

Der Pflegebetrieb des Reinigungsgeräts kann demnach ein nach einem solchen Reinigungsbetrieb des Reinigungsgeräts folgender Pflegeprozess sein, in welchem die gereinigten medizinischen Instrumente mit dem Pflegemittel gepflegt werden. Dieser Pflegebetrieb wird im Folgenden auch als Pflegeprozess, Pflegeanwendung oder Instrumentenpflege bezeichnet. Das Pflegemittel kann beispielsweise ein Pflegeöl, kurz Öl, sein. Andere medizinische Instrumente erfordern keine derartige Pflege mit einem Pflegemittel. Bei den medizinischen Instrumenten kann es sich beispielsweise um Dentalpflegeinstrumente wie beispielsweise Hand- und Winkelstücke, Dentalturbinen, Spiegel und zusätzlich oder alternativ Haken handeln. Hand- und Winkelstücke sowie Dentalturbinen werden typischerweise einem Pflegeprozess unterzogen, wobei für Spiegel oder Haken kein Pflegeprozess vorgesehen ist. Das Reinigungsgerät kann dabei als ein thermischer Desinfektor ausgeführt sein. Das hier vorgestellte Reinigungsgerät ermöglicht nun vorteilhafterweise, dass während des Pflegebetriebs medizinischer Instrumente ein bestimmter Bereich, hier der zweite Reinigungsbereich, von dem Pflegemittel ausgespart bleibt, sodass lediglich jene zu pflegende medizinische Instrumente in dem ersten Reinigungsbereich mit dem Pflegemittel kontaktiert werden. Die Aufnahmeeinrichtung kann eine mit der Schnittstelle gekoppelte oder koppelbare Verteilkammer zum Ausgeben des Pflegemittels in die medizinischen Instrumente aufweisen, wobei die Verteilkammer zumindest eine Aufnahme aufweisen kann, an die eines der medizinischen Instrumente anschließbar ist. Ferner kann die Aufnahmeeinrichtung über eine weitere Schnittstelle mit einem Druckluftversorgungsanschluss gekoppelt oder koppelbar sein, der ausgebildet ist, um eine Druckluft zu der Verteilkammer zu leiten, um beispielsweise nach dem Pflegebetrieb ein Ausblasen des Pflegemittels aus dem medizinischen Instrument zu ermöglichen. Das Abschirmmedium kann beispielsweise Wasser und zusätzlich oder alternativ Druckluft aufweisen, beispielsweise kann das Abschirmmedium in Form einer Wasser- oder Luftbarriere von der Abschirmeinrichtung erzeugbar sein. In einer vorteilhaften Ausführungsform kann die Abschirmeinrichtung beispielsweise ausgebildet sein, um das Abschirmmedium als eine senkrecht wirkende Wasser- oder Luftbarriere zwischen dem ersten Reinigungsbereich und dem zweiten Reinigungsbereich zu erzeugen. Als senkrecht ist eine in Richtung der Schwerkraft wirkende Richtung zu verstehen.

Die Abschirmeinrichtung kann eine Düseneinrichtung mit zumindest einer Düse zum Ausgeben des Abschirmmediums in den Reinigungsraum aufweisen. Mittels einer solchen Düse ist eine gezielte Ausgabe des Abschirmmediums ermöglicht.

Gemäß einer Ausführungsform kann die Düse als eine Flachstrahldüse ausgeformt sein, die ausgebildet ist, um das Abschirmmedium in einem flachen Strahl zur Erzeugung einer Trennwand zwischen dem ersten Reinigungsbereich und dem zweiten Reinigungsbereich auszugeben. Die Flachstrahldüse kann beispielsweise ausgeformt sein, um das Abschirmmedium in Form von Wasser in den Reinigungsraum auszugeben, beispielsweise kann hierbei ein Sprühbild des aus der Flachstrahldüse ausgegebenen Wassers trichterförmig ausgeformt sein. Das trichterförmige Sprühbild kann beispielsweise einen sich von der Düse weg aufweitenden Trichter ausformen. Die Düseneinrichtung kann auch eine Mehrzahl der Düsen aufweisen, die beispielsweise in Form von mehreren Flachstrahldüsen ausgeformt sein können. Die Flachstrahldüsen können fluchtend zueinander und zusätzlich oder alternativ gleichmäßig zueinander beabstandet angeordnet sein und beispielsweise mehrere trichterförmige und zusätzlich oder alternativ sich überlappende Sprühbilder erzeugen, die je einen Trennwandabschnitt der Trennwand ausformen. Die Düse kann aber auch als eine Druckluftdüse ausgeformt sein, die ausgeformt ist, um das Abschirmmedium in Form von Druckluft in den Reinigungsraum auszugeben. Beispielsweise kann hierbei ein Sprühbild der aus der Druckluftdüse ausgegebenen Druckluft rechteckig ausgeformt sein, um eine durchgehende Trennwand auszubilden. Eine derartig erzeugbare Trennwand aus Wasser und zusätzlich oder alternativ Druckluft kann schnell und einfach erzeugbar und auch wieder entfernbar sein.

Es ist weiterhin von Vorteil, wenn die Düseneinrichtung eine Verteilleiste umfasst, die ausgebildet ist, um während des Ausgebens des Abschirmmediums in den Reinigungsraum das Abschirmmedium auf zumindest die Düse oder eine Mehrzahl von Düsen der Düseneinrichtung zu verteilen. So kann sichergestellt werden, dass ein gewünschtes Sprühbild erzeugt wird. Beispielsweise können mittels der Verteilleiste mehrere der Düsen gleichmäßig mit Wasser und zusätzlich oder alternativ Druckluft beaufschlagt werden.

Das Reinigungsgerät kann ferner einen Korb und zusätzlich oder alternativ einen Instrumentenpflegeeinsatz zur Aufnahme der medizinischen Instrumente in den Reinigungsraum aufweisen, wobei die Abschirmeinrichtung in oder an dem Korb und zusätzlich oder alternativ Instrumentenpflegeeinsatz angeordnet ist. Der Instrumentenpflegeeinsatz kann ein Dentalpflegeeinsatz sein, der zur Aufnahme in den Korb ausgeformt sein kann. Die Abschirmeinrichtung kann durch ein Einschieben des Korbes mit der beispielsweise in einer Reinigungsraumrückwand des Reinigungsgeräts befindlichen Koppelstelle verbindbar sein. Ein solcher Korb und zusätzlich oder alternativ Instrumentenpflegeeinsatz ermöglicht die Aufnahme und Positionierung von medizinischen Instrumenten in dem ersten Reinigungsbereich und/oder zweiten Reinigungsbereich.

Die Abschirmmediumsleitung kann in Form einer Wasserleitung ausgeformt sein und zusätzlich oder alternativ kann das Reinigungsgerät ein Wassereinlaufventil aufweisen, wobei ein erstes Ende der Wasserleitung über die Koppelstelle fluidisch mit der Abschirmeinrichtung gekoppelt oder koppelbar ist und zusätzlich oder alternativ ein zweites Ende der Wasserleitung fluidisch mit dem Wassereinlaufventil zum Zuführen des Abschirmmediums in Form von Wasser in die Wasserleitung gekoppelt oder koppelbar ist. Über ein derartiges Wassereinlaufventil und eine derartige Wasserleitung kann vorteilhafterweise Wasser zur Erzeugung der Trennwand in dem Reinigungsraum bereitgestellt und zu der Abschirmeinrichtung gefördert werden. Bei dem Wasser kann es sich um sogenanntes VE-Wasser, also vollentsalztes Wasser handeln, das auch als demineralisiertes Wasser bezeichnet werden kann.

Die Abschirmmediumsleitung kann aber auch in Form einer Druckluftleitung ausgeformt sein, wobei ein erstes Ende der Druckluftleitung über die Koppelstelle fluidisch mit der Abschirmeinrichtung gekoppelt oder koppelbar ist und zusätzlich oder alternativ ein zweites Ende der Druckluftleitung fluidisch mit einer Drucklufteinrichtung für das Reinigungsgerät gekoppelt oder koppelbar ist, um das Abschirmmedium in Form von Druckluft in die Druckluftleitung zu leiten. Die Drucklufteinrichtung kann einen Druckluftversorgungsanschluss zum Zuführen von Druckluft in eine Leitung für Druckluft und zusätzlich oder alternativ einen Filter und zusätzlich oder alternativ ein Druckregelventil und zusätzlich oder alternativ ein Druckluftventil und zusätzlich oder alternativ ein Spülventil aufweisen. Der Filter kann ausgeformt sein, um die Druckluft zu reinigen, um beispielsweise zu verhindern, dass Keime über diesen Weg in das Reinigungsgerät gelangen. Das Druckregelventil kann ausgebildet sein, um einen Druck der Druckluft zu regeln, beispielsweise um einen stetigen Druck zu erzeugen. Das Druckluftventil kann ausgebildet sein, um die Druckluft entweder zu dem Spülventil zu leiten, oder aber eine Entlüftung eines zwischen dem Druckluftventil und dem Spülventil liegenden Leitungsabschnitts zu ermöglichen. Das Spülventil kann ausgebildet sein, um schaltbar entweder den Druckluftversorgungsanschluss oder eine Reinigungsmittelleitung über die weitere Schnittstelle mit der Aufnahmeeinrichtung zu verbinden. Die Drucklufteinrichtung kann ausgebildet sein, um die Aufnahmeeinrichtung mit Druckluft zu versorgen, um beispielsweise nach dem Pflegeprozess die an der Aufnahmeeinrichtung gekoppelten medizinischen Instrumente auszublasen.

Es ist weiterhin von Vorteil, wenn das Reinigungsgerät gemäß einer Ausführungsform ferner die Drucklufteinrichtung zum Bereitstellen der Druckluft aufweist. So kann auch die Aufnahmeeinrichtung mit Druckluft versorgt werden.

Das zweite Ende der Druckluftleitung kann zwischen dem Druckregelventil der Drucklufteinrichtung und dem Druckluftventil der Drucklufteinrichtung fluidisch mit der Drucklufteinrichtung gekoppelt oder koppelbar sein. So kann die Druckluftleitung als ein Abzweigpfad von der Drucklufteinrichtung dazu genutzt werden, Druckluft auch zu der Abschirmeinrichtung zu leiten.

Die Druckluftleitung kann ferner ein Abschirmventil zum Öffnen und zusätzlich oder alternativ Schließen der Druckluftleitung aufweisen. So kann die Druckluft entweder nur für die Aufnahmeeinrichtung bereitgestellt werden oder aber für die Aufnahmeeinrichtung und die Abschirmeinrichtung zusammen bereitgestellt werden.

Es ist weiterhin von Vorteil, wenn das Reinigungsgerät gemäß einer Ausführungsform ferner eine Steuereinrichtung aufweist, die ausgebildet ist, um ein Aktivierungssignal auszugeben, das ausgebildet ist, um einen Betrieb der Abschirmeinrichtung ansprechend auf den Pflegebetrieb zu aktivieren, um das Abschirmmedium vor und zusätzlich oder alternativ während des Pflegebetriebs auszugeben. Die Steuereinrichtung kann ferner ausgebildet sein, um ein weiteres Aktivierungssignal auszugeben, das ausgebildet ist, um einen Betrieb der Abschirmeinrichtung ansprechend auf einen nach dem Pflegebetrieb durchgeführten Ausblasbetrieb zu aktivieren, um das Abschirmmedium vor und zusätzlich oder alternativ während des Ausblasbetriebs des an der Aufnahmeeinrichtung angeordneten medizinischen Instruments auszugeben. So kann sichergestellt werden, dass während des Pflegebetriebs und optional auch während des Ausblasbetriebs das Abschirmmedium automatisiert ausgegeben wird, um den zweiten Reinigungsbereich vor dem Pflegemittel zu schützen.

Das Reinigungsgerät kann ferner eine Erkenneinrichtung aufweisen, die ausgebildet ist, um ein Erkennsignal bereitzustellen, das ein Erkennen eines in dem zweiten Reinigungsbereich des Reinigungsraums angeordneten medizinischen Instruments repräsentiert, wobei die Steuereinrichtung ausgebildet ist, um das Aktivierungssignal unter Verwendung des Erkennsignals auszugeben. Das Erkennsignal kann durch eine Nutzereingabe an beispielsweise dem Reinigungsgerät erzeugbar sein. So kann das Abschirmmedium beispielsweise nur dann automatisiert ausgegeben werden, wenn auch ein medizinisches Instrument in dem zweiten Reinigungsbereich angeordnet ist.

Gemäß einer Ausführungsform kann das Reinigungsgerät ferner eine Pflegemittelbereitstellungseinheit zur Bereitstellung des Pflegemittels aufweisen, wobei die Pflegemittelbereitstellungseinheit ausgebildet ist, um unter Verwendung eines Pflegemittelsignals das Pflegemittel über die Pflegemittelleitung zu der in dem Reinigungsraum angeordneten Aufnahmeeinrichtung zu fördern. So kann die Bereitstellung des Pflegemittels automatisiert erfolgen.

Das Reinigungsgerät kann auch ein Pflegemittelventil aufweisen, das ausgebildet ist, um die Pflegemittelbereitstellungseinheit schaltbar mit der Aufnahmeeinrichtung zu verbinden. Ein solches Pflegemittelventil kann beispielsweise für eine bestimmte Öffnungszeit oder Öffnungsdauer geöffnet werden, um in einem gewünschten Zeitraum beispielsweise eine gewünschte Menge an Pflegemittel während beispielsweise des Pflegebetriebs bereitzustellen.

Ein Verfahren zum Betreiben eines Reinigungsgeräts in einer der vorangehend beschriebenen Varianten weist einen Schritt des Durchführens und einen Schritt des Ausgebens auf. Im Schritt des Durchführens wird der Pflegebetrieb durchgeführt, um das in dem Reinigungsraum aufgenommene medizinische Instrument mit dem Pflegemittel zu pflegen. Im Schritt des Ausgebens wird das Abschirmmediums ausgegeben, um den ersten Reinigungsbereich des Reinigungsraums des Reinigungsgeräts von dem zweiten Reinigungsbereich des Reinigungsraums des Reinigungsgeräts zu separieren, um während des Pflegebetriebs ein Übertragen des Pflegemittels von dem ersten Reinigungsbereich in den zweiten Reinigungsbereich zu verhindern. Auch durch diese Ausführungsvariante der Erfindung in Form eines Verfahrens kann die der Erfindung zugrunde liegende Aufgabe schnell und effizient gelöst werden.

Das Verfahren kann ferner einen Schritt des Erkennens vor dem Schritt des Ausgebens aufweisen, wobei im Schritt des Erkennens zumindest ein in dem zweiten Reinigungsbereich angeordnetes medizinisches Instrument erkannt wird, wobei der Schritt des Ausgebens ansprechend auf den Schritt des Erkennens durchgeführt wird.

Der hier beschriebene Ansatz kann anhand eines Haushaltgeräts oder entsprechend im Zusammenhang mit einem gewerblichen oder professionellen Gerät, beispielsweise einem medizinischen Gerät, wie einem Reinigungs- oder Desinfektionsgerät, einem Kleinsterilisator, einem Großraumdesinfektor oder einer Container-Waschanlage eingesetzt werden.

Ein Ausführungsbeispiel der Erfindung ist in den Zeichnungen rein schematisch dargestellt und wird nachfolgend näher beschrieben. Es zeigt
- Figur 1: eine schematische Darstellung eines Dental-Reinigungs- und Desinfektionsgeräts mit Pflegefunktion gemäß einem Ausführungsbeispiel;
- Figur 2: eine schematische Darstellung eines Reinigungsgeräts gemäß einem Ausführungsbeispiel;
- Figur 3: eine schematische Darstellung eines Reinigungsgeräts gemäß einem Ausführungsbeispiel;
- Figur 4: eine perspektivische Darstellung einer Abschirmeinrichtung eines Reinigungsgeräts gemäß einem Ausführungsbeispiel;
- Figur 5: eine perspektivische Darstellung einer Abschirmeinrichtung eines Reinigungsgeräts gemäß einem Ausführungsbeispiel;
- Figur 6: eine perspektivische Darstellung einer Abschirmeinrichtung eines Reinigungsgeräts gemäß einem Ausführungsbeispiel;
- Figur 7: eine perspektivische Darstellung einer Abschirmeinrichtung eines Reinigungsgeräts gemäß einem Ausführungsbeispiel;
- Figur 8: eine perspektivische Darstellung einer Abschirmeinrichtung eines Reinigungsgeräts gemäß einem Ausführungsbeispiel;
- Figur 9: eine Frontalansicht einer Abschirmeinrichtung eines Reinigungsgeräts gemäß einem Ausführungsbeispiel; und
- Figur 10: ein Ablaufdiagramm eines Ausführungsbeispiels eines Verfahrens zum Betreiben eines Reinigungsgeräts.

In der nachfolgenden Beschreibung günstiger Ausführungsbeispiele des vorliegenden Ansatzes werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

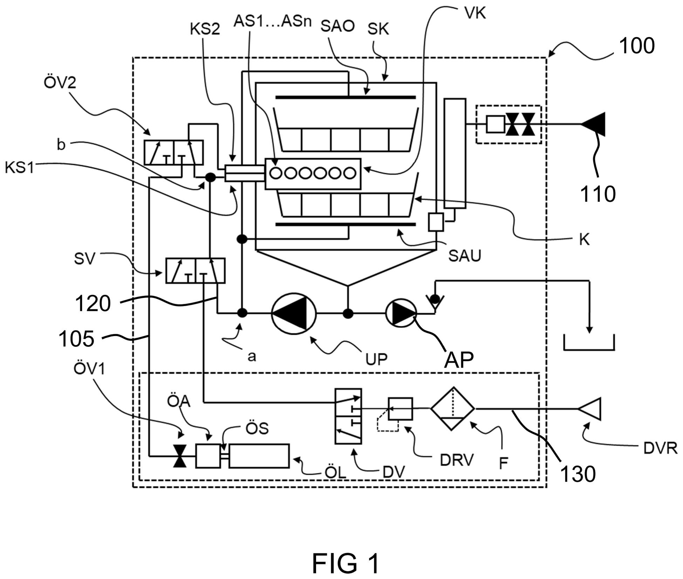

Figur 1 zeigt eine schematische Darstellung eines Dental-Reinigungs- und Desinfektionsgeräts 100 mit Pflegefunktion. Das Dental-Reinigungs- und Desinfektionsgerät 100 umfasst einen Reinigungsraum SK, in dem medizinische Instrumente gereinigt werden können. In dem Reinigungsraum SK sind beispielhaft ein Sprüharm SAO, ein weiterer Sprüharm SAU, eine Verteilkammer VK mit einer Mehrzahl an Aufnahmen AS und zumindest ein Korb K angeordnet. Zu reinigende Instrumente können an die Mehrzahl an Aufnahmen AS angekoppelt werden und während eines Reinigungsprozesses von außen und innen gereinigt werden und zumindest von innen gepflegt werden.

Das Dental-Reinigungs- und Desinfektionsgerät 100 weist eine Pflegemittelleitung 105 auf, die ausgeformt ist, um ein Pflegemittel von einer Pflegemittelbereitstellungseinheit ÖL über ein Pflegemittelventil ÖV2 an eine Schnittstelle KS2 des Reinigungsraums SK in die Verteilkammer VK zu leiten. Das Pflegemittelventil ÖV2 ist beispielsweise ausgebildet, um die Pflegemittelbereitstellungseinheit ÖL schaltbar mit der Schnittstelle KS2 zu der Verteilkammer VK zu verbinden.

Weiterhin weist das Dental-Reinigungs- und Desinfektionsgerät 100 einen Reinigungsmittelanschluss 110 auf, der eine Aufnahme von Reinigungsmittel in das Dental-Reinigungs- und Desinfektionsgerät 100 ermöglicht. Das Reinigungsmittel wird beispielsweise unter Verwendung einer Reinigungsmittelfördereinrichtung UP in den Reinigungsraum SK gefördert, dort verteilt und optional umgewälzt.

Das Dental-Reinigungs- und Desinfektionsgerät 100 umfasst einen Druckluftversorgungsanschluss DVR, der ausgebildet ist, um Druckluft zu der Verteilkammer VK zu leiten, um beispielsweise Rückstände des Reinigungsmittels oder Pflegemittels aus entsprechenden Komponenten der zu reinigen Instrumente mittels eines Druckluftstoßes auszublasen.

Eine mit der Reinigungsmittelfördereinrichtung UP verbundene Reinigungsmittelleitung 120 und eine mit dem Druckluftversorgungsanschluss DVR verbundene Leitung 130 für Druckluft sind über ein Spülmittelventil SV schaltbar mit der Schnittstelle KS2 und/oder einer weiteren Schnittstelle KS1 verbunden, sodass über das Spülmittelventil SV eingestellt werden kann, ob zu der Verteilkammer VK Reinigungsmittel oder Druckluft geführt wird.

Das Dental-Reinigungs- und Desinfektionsgerät 100 ist als ein Dentalpflegegerät ausgeformt, welches medizinische Instrumente reinigt, desinfiziert und pflegt. Die medizinischen Instrumente werden dabei in dem Reinigungsraum SK beispielsweise an die Verteilkammer VK aufgenommen. Gereinigt werden die medizinischen Instrumente durch von dem Reinigungsmittelanschluss 110 bereitgestelltem Reinigungsmittel. Das Reinigungsmittel wird beispielsweise von der Reinigungsmittelfördereinrichtung UP über eine Abzweigstelle a, dem Spülmittelventil SV und einer weiteren Abzweigstelle b zu der weiteren Schnittstelle KS1 geleitet, oder von der Abzweigstelle a zu den Sprüharmen SAO, SAU geleitet. In dem Reinigungsraum SK wird das Reinigungsmittel dann von der weiteren Schnittstelle KS1 in die Verteilkammer VK. Die Verteilkammer VK ist dabei zum Ausgeben eines eingeleiteten Fluids, also der Druckluft, des Reinigungsmediums oder des Pflegemittels, in die angekoppelten medizinischen Instrumente ausgeformt. Nach einem Reinigungsvorgang ist das verwendete Reinigungsmittel durch eine Ablaufpumpe AP aus dem Dental-Reinigungs- und Desinfektionsgerät 100 ableitbar oder ausförderbar.

Das Pflegemittel wird beispielsweise nach einem Reinigungsvorgang von der Pflegemittelbereitstellungseinheit ÖL in die Pflegemittelleitung 105 gegeben oder gefördert. Die Pflegemittelbereitstellungseinheit ÖL ist dabei beispielsweise als eine Ölkartusche oder eine Öldose mit Treibgas ausgeformt. Dabei wird das Pflegemittel beispielsweise erst von einem Ölstößel ÖS in eine Ölkartuschenadaption ÖA gegeben, welche schließlich das Pflegemittel ausgibt. Um zu verhindern, dass das Pflegemittel zurück in die Ölkartuschenadaption ÖA fließt, ist ein Ölventil ÖV1 in der Pflegemittelleitung 105 angeordnet. Das Pflegemittel wird unter Verwendung der Pflegemittelleitung 105 zu dem Pflegemittelventil ÖV2 und von dort zu der Schnittstelle KS2 geleitet.

Der Druckluftversorgungsanschluss DVR ist ausgebildet, um Druckluft in die Leitung 130 des Dental-Reinigungs- und Desinfektionsgeräts 100 zu leiten. Der Druckluftversorgungsanschluss DVR wird verwendet, um die Druckluft zu der Verteilkammer VK zu leiten. Dabei wird die Druckluft in dem Dental-Reinigungs- und Desinfektionsgerät 100 optional zuerst von einem Filter F gereinigt, um zu verhindern, dass Keime über diesen Weg in das Dental-Reinigungs- und Desinfektionsgerät 100 gelangen. Um einen stetigen Druck zu erzeugen, umfasst das Dental-Reinigungs- und Desinfektionsgerät 100 optional ein Druckregelventil DRV. Dabei wird ein Druck von beispielsweise 3 bis 5 bar durch das Druckregelventil DRV gelassen. Anschließend wird die Druckluft durch ein Druckluftventil DV geleitet. Das Druckluftventil DV ist ausgebildet, um die Druckluft entweder zu dem Spülmittelventil SV zu leiten, oder aber eine Entlüftung eines zwischen dem Druckluftventil DV und dem Spülmittelventil SV liegenden Leitungsabschnitts zu ermöglichen.

Das Spülmittelventil SV wird verwendet, um entweder den Druckluftversorgungsanschluss DVR oder die Reinigungsmittelleitung 120 mit der Verteilkammer VK schaltbar zu verbinden. Die Pflegemittelleitung 105, die Leitung 130 und die Reinigungsmittelleitung 120 sind hier als Versorgungsleitungen ausgeformt, welche unterschiedliche Fluide zu der Verteilkammer VK leiten.

Das Pflegemittel wird durch die Schnittstelle KS2 zu der Verteilkammer VK geleitet. Das Reinigungsmittel und auch die Druckluft werden durch die weitere Schnittstelle KS1 und/oder die Schnittstelle KS2 in den Reinigungsraum SK und/oder zu der Verteilkammer VK geleitet. Die Schnittstelle KS2 und die weitere Schnittstelle KS1 sind dabei Koppelstellen für die Verteilkammer VK. Die Verteilkammer VK weist eine Mehrzahl an Aufnahmen AS auf. Die Mehrzahl an Aufnahmen AS ist beispielsweise als eine Mehrzahl an Anschlusspositionen ausgeformt. An der Mehrzahl an Aufnahmen AS kann hier jeweils ein medizinisches Instrument angeschlossen werden, um dieses von innen zu reinigen und zu pflegen. Um die an die Verteilkammer VK angeschlossenen medizinischen Instrumente zu halten oder weitere medizinische Instrumente aufzunehmen, ist der Reinigungsraum SK optional mit dem Korb K ausgestattet. Das Dental-Reinigungs- und Desinfektionsgerät 100 umfasst hier lediglich beispielhaft auch einen weiteren Korb.

Vorteilhafterweise können in dem Reinigungsgerät WD gleichzeitig sowohl mit dem Pflegemittel zu behandelnde Instrumente als auch nicht mit dem Pflegemittel zu behandelnde Instrumente gereinigt werden. Dazu werden diese unterschiedlich zu behandelnden Instrumente getrennt voneinander in unterschiedlichen Reinigungsbereichen des Reinigungsraums SK angeordnet. Wie anhand der nachfolgenden Figuren näher erläutert wird ein Abschirmmedium verwendet, um die unterschiedlichen Reinigungsbereiche während des Anwendens des Pflegemittels so zu separieren, dass die nicht mit dem Pflegemittel zu behandelnden Instrumente nicht mit dem Pflegemittel in Berührung kommen, während die mit dem Pflegemittel zu behandelnde Instrumente mit dem Pflegemittel behandelt werden. Beispielsweise wird eine solche Trennung durch einen Wasservorhang oder einen Luftvorhang realisiert, der zwischen den unterschiedlichen Reinigungsbereichen zumindest während des Zeitraums aufgebaut wird, während dem ein unerwünschter Kontakt der nicht mit dem Pflegemittel zu behandelnden Instrumente möglich wäre.

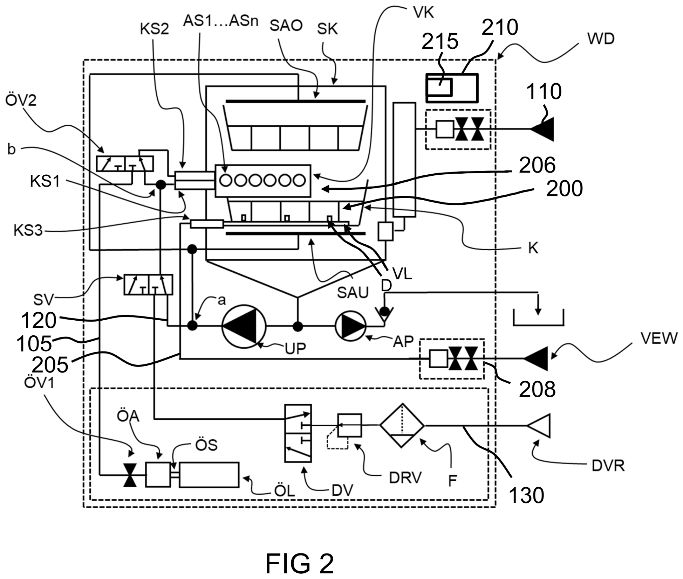

Figur 2 zeigt eine schematische Darstellung eines Reinigungsgeräts WD gemäß einem Ausführungsbeispiel. Dabei kann es sich um das in Figur 1 beschriebene Dental-Reinigungs- und Desinfektionsgerät handeln, mit dem Unterschied, dass in Figur 2 ein Ausführungsbeispiel einer Abschirmeinrichtung 200 dargestellt ist, die ausgeformt ist, um unter Verwendung eines Abschirmmediums eine Abschirmung unterschiedlicher Reinigungsbereiche des Reinigungsraums SK zu gewährleisten. Dazu ist die Abschirmeinrichtung 200 mit einer Abschirmmediumsleitung 205 zum Leiten des Abschirmmediums gekoppelt.

Lediglich optional umfasst das Reinigungsgerät WD gemäß diesem Ausführungsbeispiel die in Figur 1 beschriebenen Merkmale a, b, AP, AS, DRV, DV, DVR, F, K, KS1, SAO, SAU, SV, UP, VK, ÖA, ÖL, ÖS, ÖV1, ÖV2, 110, 120, 130. Gemäß einem alternativen Ausführungsbeispiel umfasst das Reinigungsgerät WD lediglich den Reinigungsraum SK, die Pflegemittelleitung 105, die Abschirmeinrichtung 200 und die Abschirmmediumsleitung 205 und keines oder optional eines oder nur einzelne der Merkmale a, b, AP, AS, DRV, DV, DVR, F, K, KS1, SAO, SAU, SV, UP, VK, ÖA, ÖL, ÖS, ÖV1, ÖV2, 110, 120, 130.

Der Reinigungsraum SK weist einen ersten Reinigungsbereich auf, in dem eine Aufnahmeeinrichtung 206 zum Aufnehmen zumindest eines der medizinischen Instrumente angeordnet ist. Die Aufnahmeeinrichtung 206 ist gemäß diesem Ausführungsbeispiel als die Verteilkammer VK ausgeformt. Die Pflegemittelleitung 105 ist dazu ausgebildet, um das Pflegemittel an die Schnittstelle KS2 zu der Aufnahmeeinrichtung 206 zu leiten, um das in oder an der Aufnahmeeinrichtung 206 aufgenommene medizinische Instrument während eines Pflegebetriebs mit dem Pflegemittel zu pflegen. Die Abschirmeinrichtung 200 ist innerhalb des Reinigungsraums SK angeordnet oder anordenbar und ist ausgebildet, um ein Abschirmmedium auszugeben, um den ersten Reinigungsbereich von einem sich von dem ersten Reinigungsbereich unterscheidenden zweiten Reinigungsbereich des Reinigungsraums SK zu separieren, um während des Pflegebetriebs ein Übertragen des Pflegemittels von dem ersten Reinigungsbereich in den zweiten Reinigungsbereich zu verhindern. Die Abschirmmediumsleitung 205 ist ausgebildet, um das Abschirmmedium an eine Koppelstelle KS3 zu der Abschirmeinrichtung zu leiten.

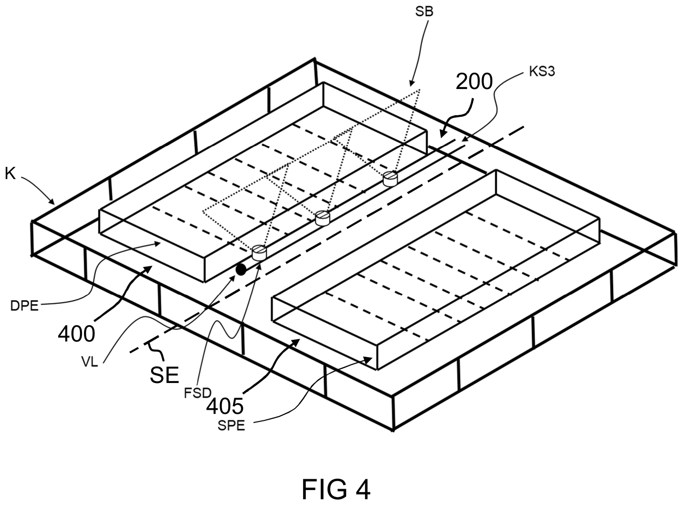

Einige medizinische Instrumente erfordern nach dem Reinigungsbetrieb, in welchem die medizinischen Instrumente gereinigt werden, eine Pflege mit Pflegemittel. Der Pflegebetrieb des Reinigungsgeräts WD ist demnach gemäß diesem Ausführungsbeispiel ein nach einem solchen Reinigungsbetrieb des Reinigungsgeräts WD folgender Pflegeprozess, in welchem die gereinigten medizinischen Instrumente mit dem Pflegemittel gepflegt und&/oder aufbereitet werden. Das Pflegemittel ist gemäß diesem Ausführungsbeispiel als ein Pflegeöl ausgeformt. Andere medizinische Instrumente erfordern keine derartige Pflege mit einem Pflegemittel. Bei den medizinischen Instrumenten handelt es sich beispielsweise um Dentalpflegeinstrumente wie beispielsweise Hand- und Winkelstücke, Spiegel und zusätzlich oder alternativ Haken. Hand- und Winkelstücke werden typischerweise einem Pflegeprozess unterzogen, wobei für Spiegel oder Haken kein Pflegeprozess mit Pflegeöl vorgesehen ist. Das hier vorgestellte Reinigungsgerät WD ermöglicht nun vorteilhafterweise, dass während des Pflegebetriebs medizinischer Instrumente ein bestimmter Bereich, hier der zweite Reinigungsbereich, von dem Pflegemittel ausgespart bleibt, sodass lediglich jene zu pflegende medizinische Instrumente in dem ersten Reinigungsbereich mit dem Pflegemittel kontaktiert werden. Der erste Reinigungsbereich und der zweite Reinigungsbereich sind in den Figuren 4, 5, 6 und 9 gezeigt. Die Aufnahmeeinrichtung 206 weist gemäß diesem Ausführungsbeispiel die mit der Schnittstelle KS2 gekoppelte oder koppelbare Verteilkammer VK zum Ausgeben des Pflegemittels in die medizinischen Instrumente auf. Ferner ist die Aufnahmeeinrichtung 206 gemäß diesem Ausführungsbeispiel über die weitere Schnittstelle KS1 mit dem Druckluftversorgungsanschluss DVR gekoppelt, der ausgebildet ist, um Druckluft zu der Verteilkammer VK zu leiten, um beispielsweise nach dem Pflegebetrieb ein Ausblasen des Pflegemittels aus dem medizinischen Instrument zu ermöglichen. Das Abschirmmedium ist gemäß diesem Ausführungsbeispiel als Wasser oder gemäß einem alternativen Ausführungsbeispiel als Druckluft ausgeformt. Beispielsweise ist die Abschirmeinrichtung 200 gemäß diesem Ausführungsbeispiel ausgebildet, um das Abschirmmedium in Form einer Wasserbarriere zu erzeugen, um in dem Reinigungsraum SK eine Trennebene aus Wasser herzustellen.

Die Abschirmeinrichtung 200 weist gemäß diesem Ausführungsbeispiel eine Düseneinrichtung mit zumindest einer Düse D zum Ausgeben des Abschirmmediums in den Reinigungsraum SK auf. Lediglich optional weist die Düseneinrichtung gemäß diesem Ausführungsbeispiel drei der Düsen D auf, die beispielsweise fluchtend und/oder gleichmäßig zueinander beabstandet angeordnet sind. Gemäß einem alternativen Ausführungsbeispiel weist die Düseneinrichtung zwei oder mehr als drei Düsen D auf. Die Düseneinrichtung umfasst gemäß diesem Ausführungsbeispiel eine Verteilleiste VL, die ausgebildet ist, um während des Ausgebens des Abschirmmediums in den Reinigungsraum SK das Abschirmmedium auf zumindest die Düse D oder mehrere Düsen D der Düseneinrichtung zu verteilen. Die Verteilleiste VL ist gemäß diesem Ausführungsbeispiel gerade und/oder rohrförmig ausgeformt.

Das Reinigungsgerät WD weist gemäß diesem Ausführungsbeispiel ferner den Korb K und zusätzlich oder alternativ einen Instrumentenpflegeeinsatz zur Aufnahme der medizinischen Instrumente in den Reinigungsraum SK auf, wobei die Abschirmeinrichtung 200 in oder an dem Korb K und zusätzlich oder alternativ in oder an dem Instrumentenpflegeeinsatz angeordnet ist. Die Abschirmeinrichtung 200 ist gemäß diesem Ausführungsbeispiel durch ein Einschieben des Korbes K mit der gemäß diesem Ausführungsbeispiel in einer Reinigungsraumrückwand des Reinigungsgeräts WD befindlichen Koppelstelle KS3 verbindbar.

Die Abschirmmediumsleitung 205 ist gemäß diesem Ausführungsbeispiel in Form einer Wasserleitung ausgeformt. Das Reinigungsgerät WD weist ferner gemäß diesem Ausführungsbeispiel ein Wassereinlaufventil VEW auf, wobei ein erstes Ende der Wasserleitung über die Koppelstelle KS3 fluidisch mit der Abschirmeinrichtung 200 gekoppelt oder koppelbar ist und zusätzlich oder alternativ ein zweites Ende der Wasserleitung fluidisch mit dem Wassereinlaufventil VEW zum Zuführen des Abschirmmediums in Form von Wasser in die Wasserleitung gekoppelt oder koppelbar ist. Bei dem Wasser handelt es sich gemäß diesem Ausführungsbeispiel um sogenanntes VE-Wasser, also vollentsalztes Wasser, das auch als demineralisiertes Wasser bezeichnet werden kann. Die Wasserleitung umfasst gemäß diesem Ausführungsbeispiel ferner ein zwischen dem Wassereinlaufventil VEW und der Koppelstelle KS3 angeordnetes Wasserventil 208.

Das Reinigungsgerät WD weist gemäß diesem Ausführungsbeispiel ferner eine Steuereinrichtung 210 auf, die ausgebildet ist, um ein Aktivierungssignal auszugeben, das ausgebildet ist, um einen Betrieb der Abschirmeinrichtung 200 ansprechend auf den Pflegebetrieb zu aktivieren, um das Abschirmmedium vor und zusätzlich oder alternativ während des Pflegebetriebs auszugeben. Die Steuereinrichtung 210 ist gemäß diesem Ausführungsbeispiel ferner ausgebildet, um ein weiteres Aktivierungssignal auszugeben, das ausgebildet ist, um einen Betrieb der Abschirmeinrichtung ansprechend auf einen nach dem Pflegebetrieb durchgeführten Ausblasbetrieb mit Druckluft zu aktivieren, um das Abschirmmedium vor und zusätzlich oder alternativ während des Ausblasbetriebs des medizinischen Instruments an der Aufnahmeeinrichtung 206 auszugeben. Das Reinigungsgerät WD weist gemäß diesem Ausführungsbeispiel ferner eine Erkenneinrichtung 215 auf, die ausgebildet ist, um ein Erkennsignal bereitzustellen, das ein Erkennen eines in dem zweiten Reinigungsbereich des Reinigungsraums SK angeordneten medizinischen Instruments repräsentiert, wobei die Steuereinrichtung 210 ausgebildet ist, um das Aktivierungssignal unter Verwendung des Erkennsignals auszugeben. Das Erkennsignal ist gemäß einem Ausführungsbeispiel durch eine Nutzereingabe an beispielsweise dem Reinigungsgerät WD erzeugbar.

Die Pflegemittelbereitstellungseinheit ÖL ist gemäß diesem Ausführungsbeispiel ausgebildet, um unter Verwendung eines Pflegemittelsignals das Pflegemittel über die Pflegemittelleitung 105 zu der in dem Reinigungsraum SK angeordneten Aufnahmeeinrichtung 206 zu fördern.

Das hier vorgestellte Reinigungsgerät WD kann auch als ein "RDG mit Pflegefunktion und Instrumentenabschirmung" bezeichnet werden, wobei "RDG" für Reinigungs- und Desinfektionsgerät steht. Das Reinigungsgerät WD ermöglicht eine temporäre Abschirmung nicht zu ölender Instrumente in einem thermischen Desinfektor mit Dentalpflegefunktion.

Bei der Aufbereitung von medizinischen Instrumenten in Form von Übertragungsinstrumenten wie Turbinen und/oder Hand- und Winkelstücken kommen üblicherweise sowohl thermische Reinigungs- und Desinfektionsgeräte als auch Pflegegeräte und Autoklaven zum Einsatz. Die Pflegegeräte sind entweder zur ausschließlichen Pflege der Instrumente gedacht, oder sie ermöglichen zusätzlich eine chemische Innen- und Außenreinigung der Instrumente. Eine Kombination der Funktionen in einem Dental-RDG, setzt voraus, dass bei einer gemischten Beladung, bestehend aus beispielsweise zu pflegenden Hand- und Winkelstücken in einem speziellen Korbeinsatz und einfachen Dentalinstrumenten, wie beispielsweise Spiegeln, Haken, etc., welche beispielsweise in ein Reinigungssieb eingelegt werden, nach dem vollständigen Durchlaufen des Aufbereitungs- und Pflegeprozesses keine Pflegeölrückstände auf den Instrumenten und im Reinigungsraum SK, der auch als Spülraum bezeichnet werden kann, verbleiben. Zwar stellen derartige Pflegeölrückstände weder ein hygienisches noch ein gesundheitliches Risiko für einen Patienten dar, dennoch ist dieser Umstand unerwünscht und trägt maßgeblich zu einer weniger positiven Wahrnehmung des Instruments bei.

Durch allein eine zeitlich geschickte Ansteuerung einer Pflegeperipherie innerhalb eines Pflege- und/oder Aufbereitungsprozesses ergeben sich zwar diverse Möglichkeiten zur grundsätzlichen Reduzierung der Ölrückstände auf den neben dem Korbeinsatz für Hand- und Winkelstücke oder Turbinen befindlichen Instrumenten, dennoch kann eine Verteilung von Pflegeöl oder ölhaltigem Nebel im Reinigungsraum SK so nicht aktiv unterbunden werden. Daher kommt die hier vorgestellte Abschirmeinrichtung 200 zum Einsatz, die eine hoch wirtschaftliche, effektive und hygienische Möglichkeit zur Ölabschirmung eines Bereichs des Reinigungsraums SK realisiert.

Das hier vorgestellte Reinigungsgerät WD ermöglicht es also, innerhalb eines RDGs für Dentalanwendungen mit Dentalinstrumentenaufbereitung mit integrierter Pflegefunktion für Hand- und Winkelstücke und Dentalturbinen, dass im selben Reinigungsraum SK parallel weiteres Dentalinstrumentarium aufbereitet werden kann, ohne, dass dieses mit Pflegemittel in Kontakt kommt. Es wird dank der Abschirmeinrichtung 200 eine ungewollte Ölverteilung oder eine Bildung von Ölsprühnebel innerhalb eines Bereichs des Reinigungsraumes SK auf hygienische und wirtschaftliche Weise vermieden. Hierbei wird die Verteilung von Öl in dem zweiten Reinigungsbereich nicht nur verringert, sondern gemäß einem Ausführungsbeispiel sogar gänzlich verhindert.

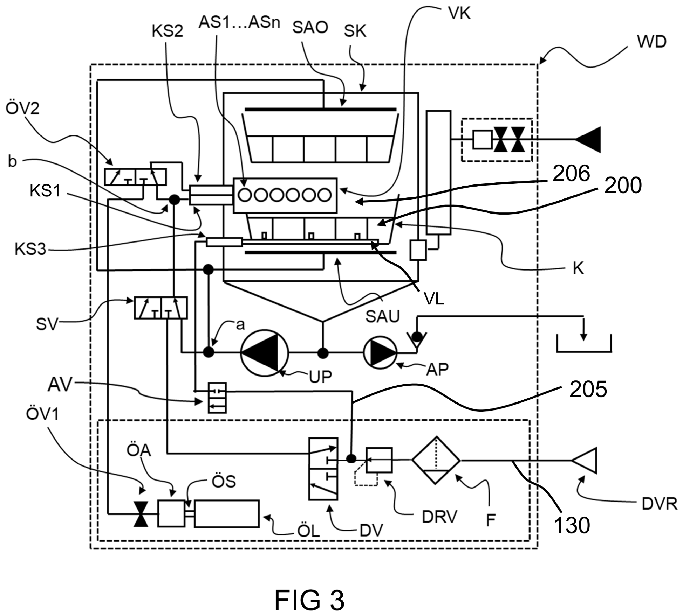

Einige Übertragungsinstrumente sollen gemäß einem Ausführungsbeispiel nach erfolgreicher Reinigung und Desinfektion mit Öl gepflegt werden, bevor sie wieder verwendet werden können. Erfolgen diese Schritte in einem Gerät und werden in diesem Gerät parallel noch weitere Dentalinstrumente aufbereitet, so ist zu verhindern, dass bei der Pflege der Hand- und Winkelstücke und/oder der Dentalturbinen, die angrenzend in einem Spülkorb platzierten Dentalinstrumente ebenfalls mit Öl in Kontakt kommen. Daher umfasst das hier vorgestellte Reinigungsgerät WD vorteilhafterweise die Abschirmeinrichtung 200, die gemäß diesem Ausführungsbeispiel im Betrieb eine temporärer Wasserabschirmung oder gemäß einem in Figur 3 beschriebenen alternativen Ausführungsbeispiel Druckluftabschirmung erzeugt, welche die Öltropfen und/oder durch das Ausblasen der Instrumente mit Druckluft entstehenden Ölnebel aufhält und/oder ablenkt und so eine unkontrollierte Verteilung im Reinigungsraum SK verhindert.

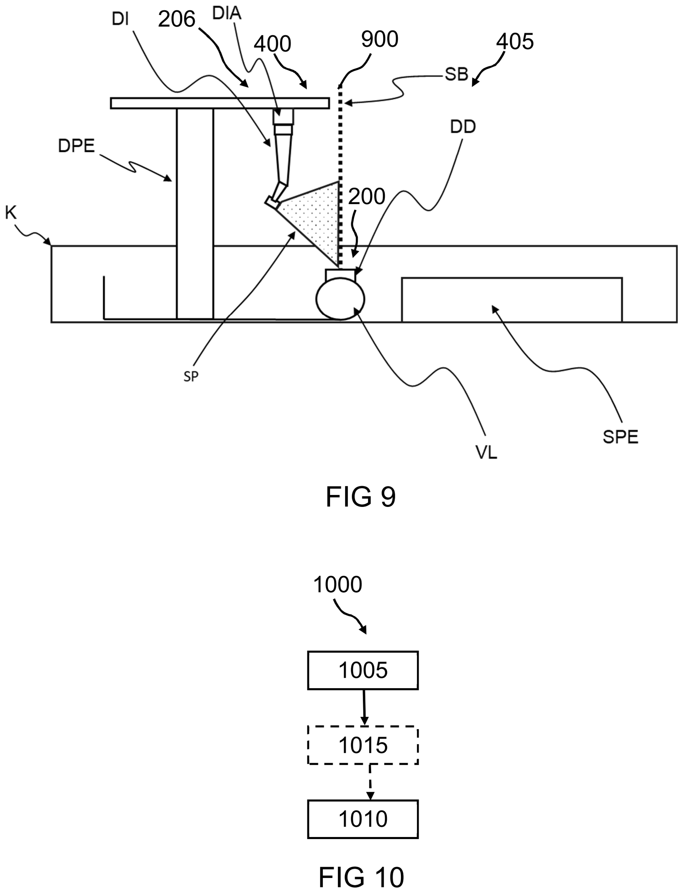

Der hier vorgestellte Ansatz umfasst grundlegend zwei Ausprägungen, welche ihrerseits auf die zukünftigen Bedürfnisse der Aufbereitungs- und Pflegeanwendungen in ihren Dimensionen skalierbar und anpassbar sind. Figur 2 zeigt eine erste Ausprägung, in der ein Einsatz einer temporären Trennebene aus Wasser zwischen den in Figur 9 gezeigten Korbsegmenten der zu pflegenden und der nicht zu pflegenden Instrumente in einem Dental-RDG mit Pflegefunktion realisiert ist. Diese Trennebene entsteht durch das Öffnen des Wassereinlaufventils VEW in Form eines Einlaufventils für VE-Wasser. Das Wasser gelangt über die Wasserleitung und die beispielsweise druckdichte Koppelstelle KS3 in die Verteilleiste VL und tritt dort gemäß einem Ausführungsbeispiel aus einer oder mehreren Düsen D aus. Eine hydraulische Verbindung zwischen der Verteilleiste VL und der Koppelstelle KS3 in der Rückwand des Reinigungsraumes SK wird gemäß diesem Ausführungsbeispiel durch das Einschieben des Korbes K in den Reinigungsraum SK hergestellt.

Die Abschirmeinrichtung 200 verhindert so das Benetzen des im Korb K vorgesehenen zweiten Reinigungsbereichs in Form eines Segmentes für allgemeine Dentalinstrumente, beispielsweise ein Spülguteinsatz in Form einer Siebschale mit Spiegeln, durch das beispielsweise an einem Dentalpflegeeinsatz in Form eines Korbeinsatzes für Hand- und Winkelstücke und/oder Dentalturbinen austretende Pflegeöl mit Hilfe der temporären Trennebene aus Wasser. Diese Trennebene wird gemäß diesem Ausführungsbeispiel durch VE-Wasser erzeugt, welches aus einer oder mehreren Düsen D austritt, die gemäß einem Ausführungsbeispiel als Flachstrahldüsen ausgeformt sind. Die Düse/n D ist/sind gemäß diesem Ausführungsbeispiel senkrecht auf der Verteilleiste VL angebracht und so orientiert, dass sich gemäß einem Ausführungsbeispiel ihr Sprühbild überschneidet, siehe Figuren 4 bis 5 und 7. Sie werden durch die Verteilleiste VL mit VE-Wasser versorgt. Die Verteilleiste VL ist gemäß einem Ausführungsbeispiel am Korb K selbst und/oder am Dentalpflegeeinsatz befestigt und wird durch das Einschieben des Korbes K mit der gemäß diesem Ausführungsbeispiel in der Reinigungsraumrückwand befindlichen Koppelstelle KS3 verbunden, die eine Korbschnittstelle sein kann. Wird nun das Wassereinlaufventil VEW für VE-Wasser geschaltet, tritt das Wasser aus den Düsen D der Verteilleiste VL aus und erzeugt eine auf die Dauer der Ventilansteuerung begrenzte Trennebene zwischen dem Spülguteinsatz und dem Dentalpflegeeinsatz, siehe auch die Figuren 4 bis 9.

Die Zeitpunkte zur Ansteuerung des Wassereinlaufventils VEW und dessen Öffnungsdauer sind abhängig vom Programmablauf des Aufbereitungs- und Pflegeprozesses. Da das Entstehen und/oder der Aufbau der temporären Trennung zwischen den Korbsegmenten eine gewisse Zeit in Anspruch nimmt, wird das Wassereinlaufventil VEW gemäß diesem Ausführungsbeispiel vor dem Öffnen des Pflegemittelventils ÖV2 und/oder des Spülventils SV mit einer Vorlaufzeit angesteuert.

Die Ansteuerung des Wassereinlaufventils VEW erfolgt gemäß diesem Ausführungsbeispiel immer dann, wenn der Dentalpflegeeinsatz und alle angeschlossenen Instrumente an den Adaptionsstellen AS1 bis ASn über die Schnittstellen KS1 und KS2 und die Ventile SV, ÖV1, ÖV2 und DV abwechselnd mit Pflegeöl und Druckluft zur Instrumentenpflege oder mit Druckluft zur Trocknung während eines Ausblasbetriebs beaufschlagt werden und sich parallel dazu nicht zu pflegende Instrumente im Reinigungsraum SK befinden. Eine Abfrage, ob dies der Fall ist, erfolgt gemäß diesem Ausführungsbeispiel vor Prozessbeginn und erfordert gemäß einem Ausführungsbeispiel eine Bestätigung oder Verneinung durch den Anwender.

Das trotz der prozessseitigen Verwendung minimaler Ölmengen und gemäß diesem Ausführungsbeispiel einer vorteilhaften konstruktiven Orientierung von Austrittsöffnungen der Hand- und Winkelstücke und/oder Dentalturbinen austretende Pflegeöl wird im entstandenen Gesamtsprühbild aller Düsen D gefangen und im Anschluss an den Programmschritt der Instrumentenpflege mit dem übrigen in einem Sammeltopf befindlichen Wasser über die Ablaufpumpe AP aus dem Reinigungsraum SK gefördert.

Eine Verölung parallel im Reinigungsraum SK befindlicher Instrumente wird durch den dort stattfindenden Pflegeprozess für Hand- und Winkelstücke und/oder Dentalturbinen auf ein absolutes Minimum reduziert oder sogar nahezu vollständig oder vollständig vermieden. So ist der Vorteil einer parallelen Mischbeladung des Reinigungsgeräts WD in Form eines Dental-RDGs ohne einen notwendigen zeitlichen Mehraufwand für eine sequenzielle Abarbeitung der Aufbereitung von Hand- und Winkelstücken und/oder Dentalturbinen und allgemeinen Dentalinstrumenten oder zusätzliche Arbeitsschritte, wie das Entnehmen der allgemeinen Dentalinstrumente aus dem Reinigungsraum SK, bevor die übrigen Instrumente gepflegt werden, vollumfänglich im Sinne des Anwendervorteils und des Gerätemehrwertes nutzbar.

Der hier vorgestellte Ansatz ist in allen RDGs einsetzbar, welche über eine Pflegefunktion für Dental- oder Schließgelenkinstrumente verfügen.

Figur 3 zeigt eine schematische Darstellung eines Reinigungsgeräts WD gemäß einem Ausführungsbeispiel. Dabei kann es sich um das in Figur 2 beschriebene Reinigungsgerät WD handeln, mit dem Unterschied, dass die Abschirmmediumsleitung 205 gemäß diesem Ausführungsbeispiel als eine Druckluftleitung ausgeformt ist. Ferner weist das Reinigungsgerät WD das in Figur 2 beschriebene Wassereinlaufventil nicht auf.

Lediglich optional weist das Reinigungsgerät WD gemäß diesem Ausführungsbeispiel eine Drucklufteinrichtung zum Bereitstellen der Druckluft auf, wobei die Drucklufteinrichtung gemäß diesem Ausführungsbeispiel die in Figur 1 beschriebenen Merkmale b, DRV, DV, DVR, F, KS1, SV, 130 umfasst.

Ein erstes Ende der Druckluftleitung ist gemäß einem Ausführungsbeispiel über die Koppelstelle KS3 fluidisch mit der Abschirmeinrichtung 200 gekoppelt oder koppelbar und zusätzlich oder alternativ ist ein zweites Ende der weiteren Druckluftleitung gemäß einem Ausführungsbeispiel fluidisch mit der Drucklufteinrichtung gekoppelt oder koppelbar, um das Abschirmmedium in Form von Druckluft in die Druckluftleitung zu leiten. Das zweite Ende der weiteren Druckluftleitung ist gemäß diesem Ausführungsbeispiel zwischen dem Druckregelventil DRV der Drucklufteinrichtung und dem Druckluftventil DV der Drucklufteinrichtung fluidisch mit der Drucklufteinrichtung gekoppelt. So ist die Druckluftleitung gemäß diesem Ausführungsbeispiel als ein Abzweigpfad von der Leitung 130 für Druckluft nutzbar, um Druckluft von dem Druckluftversorgungsanschluss DVR auch zu der Abschirmeinrichtung 200 zu leiten. Beispielsweise ist die Abschirmeinrichtung 200 gemäß diesem Ausführungsbeispiel ausgebildet, um das Abschirmmedium in Form einer Luftbarriere zu erzeugen, um in dem Reinigungsraum SK eine Trennebene aus Druckluft herzustellen.

Die Druckluftleitung weist ferner gemäß diesem Ausführungsbeispiel ein Abschirmventil AV zum Öffnen und zusätzlich oder alternativ Schließen der Druckluftleitung auf. So ist die Druckluft entweder nur für die Aufnahmeeinrichtung 206 bereitstellbar oder aber für die Aufnahmeeinrichtung 206 und die Abschirmeinrichtung 200 zusammen bereitstellbar.

In anderen Worten ausgedrückt, ist in Figur 3 eine Gerätekonfiguration des Reinigungsgeräts WD für eine temporäre Trennebene aus Druckluft dargestellt. Alle Abläufe und das Wirkprinzip sind dabei gemäß diesem Ausführungsbeispiel identisch zur in Figur 2 beschriebenen Ausprägung mit Wasser. Zur Freigabe der Druckluft, welche über den geräteseitigen Druckluftversorgungsanschluss DVR bezogen wird, wird das Abschirmventil AV angesteuert.

Figur 4 zeigt eine perspektivische Darstellung einer Abschirmeinrichtung 200 eines Reinigungsgeräts gemäß einem Ausführungsbeispiel. Dabei kann es sich um die in Figur 2 beschriebene Abschirmeinrichtung 200 handeln.

Die Abschirmeinrichtung 200 ist gemäß diesem Ausführungsbeispiel an dem Korb K und/oder dem Instrumentenpflegeeinsatz angeordnet, der gemäß diesem Ausführungsbeispiel als ein Dentalpflegeeinsatz DPE ausgeformt ist und in dem Korb K aufgenommen ist. Ebenfalls in dem Korb K angeordnet ist gemäß diesem Ausführungsbeispiel ein Spülguteinsatz SPE, in welchem die Reinigung von beispielsweise Spiegeln und/oder Haken vorgesehen ist. Eine Schnittebene SE zeigt schematisch eine Grenze zwischen dem ersten Reinigungsbereich 400 und dem zweiten Reinigungsbereich 405 des Reinigungsraums an. Der Dentalpflegeeinsatz DPE ist gemäß diesem Ausführungsbeispiel in dem ersten Reinigungsbereich 400 und der Spülguteinsatz SPE in dem zweiten Reinigungsbereich 405 angeordnet.

Gemäß diesem Ausführungsbeispiel ist die Düse als eine Flachstrahldüse FSD ausgeformt, die ausgebildet ist, um das Abschirmmedium in einem flachen Strahl zur Erzeugung einer Trennwand zwischen dem ersten Reinigungsbereich 400 und dem zweiten Reinigungsbereich 405 auszugeben. Die Flachstrahldüse FSD ist gemäß diesem Ausführungsbeispiel ausgeformt, um das Abschirmmedium in Form von Wasser in den Reinigungsraum auszugeben, beispielsweise ist hierbei ein Sprühbild SB des aus der Flachstrahldüse FSD ausgegebenen Wassers trichterförmig ausgeformt. Das trichterförmige Sprühbild SB formt beispielsweise einen sich von der Düse weg aufweitenden Trichter aus. Gemäß diesem Ausführungsbeispiel sind alle drei Düsen als derartige Flachstrahldüsen FSD ausgeformt. Die Flachstrahldüsen FSD sind gemäß diesem Ausführungsbeispiel fluchtend entlang der Verteilleiste VL angeordnet und erzeugen gemäß diesem Ausführungsbeispiel je ein trichterförmiges und/oder einander überlappende Sprühbilder SB, die je einen Trennwandabschnitt zur Erzeugung der Trennwand ausformen. Gemäß diesem Ausführungsbeispiel ist die Abschirmeinrichtung 200 ausgebildet, um das Abschirmmedium als eine senkrecht wirkende Wasserbarriere zwischen dem ersten Reinigungsbereich 400 und dem zweiten Reinigungsbereich 405 zu erzeugen. Als senkrecht ist eine in Richtung der Schwerkraft wirkende Richtung zu verstehen.

Eine der Schnittebene SE folgende Länge der Verteilleiste VL entspricht gemäß diesem Ausführungsbeispiel mehr als einer Hälfte einer Länge des Korbs K. Der Dentalpflegeeinsatz DPE belegt gemäß diesem Ausführungsbeispiel weniger als eine Hälfte einer Grundfläche des Korbs K. Der Spülguteinsatz SPE belegt gemäß diesem Ausführungsbeispiel weniger als eine andere Hälfte der Grundfläche des Korbs K.

In Figur 4 ist in anderen Worten ausgedrückt ein beispielhafter Dentalpflegeeinsatz DPE in Form eines Spülkorbeinsatzes für Hand- und Winkelstücke und/oder Dentalturbinen mit Wasserabschirmung dargestellt. Die Abschirmeinrichtung 200 erzeugt gemäß diesem Ausführungsbeispiel eine parallele Trennebene aus Wasser.

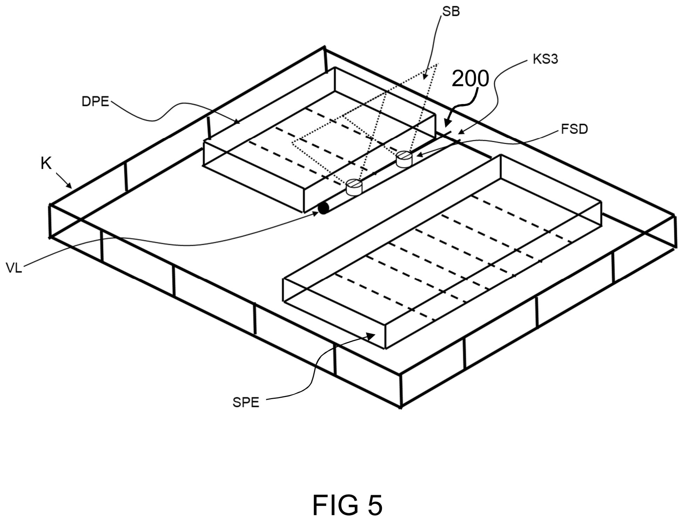

Da die Aufteilung des Korbs K, der auch als Spülkorb bezeichnet werden kann, für unterschiedliche Beladungsträger wie Dentalpflegeeinsatz DPE, Instrumentenkörbe, Spüldüseneinsätze, etc. gemäß diesem Ausführungsbeispiel mittels einer Segmentierung einer Grundfläche des Korbes K erfolgt, wird die Schnittebene SE zwischen einem Segment für den Dentalpflegeeinsatz DPE und dem angrenzenden Segment gemäß diesem Ausführungsbeispiel durch einen Wasservorhang oder gemäß einem alternativen Ausführungsbeispiel durch einen Luftvorhang getrennt. Dieser Vorhang entsteht durch eine oder mehrere gemäß diesem Ausführungsbeispiel am Dentalpflegeeinsatz DPE angebrachte Flachstrahldüsen FSD, welche im Betrieb der Abschirmeinrichtung 200 über die gemäß diesem Ausführungsbeispiel im Dentalpflegeeinsatz DPE verlegte Versorgungsleitung VL und eine Schnittstelle in der Reinigungsraumrückwand mit VE-Wasser oder alternativ mit Druckluft versorgt werden. Die Flachstrahldüse FSD oder die Flachstrahldüsen FSD sind gemäß diesem Ausführungsbeispiel so orientiert, dass sie eine senkrecht im Korb K wirkende Wasser- oder alternativ Luftbarriere entstehen lassen, in welcher sich eventuell aus den Instrumenten austretende Öltropfen verfangen oder in einen bezüglich der Benetzung anderer aufzubereitender Instrumente unkritischen Bereich abgelenkt werden und diese so nicht mehr auf die übrigen Instrumente gelangen. Die Flachstrahldüsen FSD werden gemäß diesem Ausführungsbeispiel immer dann durch das Öffnen des Wasserventils und/oder Wassereinlaufventils mit VE-Wasser oder gemäß einem alternativen Ausführungsbeispiel mit Druckluft beaufschlagt, wenn Pflegeöl durch die innenliegenden Geometrien der Hand- und Winkelstücke und/oder Turbinen geleitet wird, oder bei deren anschließender Trocknung durch das Ausblasen mit Druckluft. Die Figuren 4 und 5 zeigen unterschiedliche der in Figur 2 beschriebenen Ausprägungen mit Wasser in ihrer Umsetzung im Korb K.

Figur 5 zeigt eine perspektivische Darstellung einer Abschirmeinrichtung 200 eines Reinigungsgeräts gemäß einem Ausführungsbeispiel. Dabei kann es sich um die in Figur 4 beschriebene Abschirmeinrichtung 200 handeln, mit dem Unterschied, dass die Düseneinrichtung gemäß diesem Ausführungsbeispiel lediglich zwei der Flachstrahldüsen FSD aufweist. Eine Länge der Verteilleiste VL entspricht gemäß diesem Ausführungsbeispiel der Hälfte der Länge des Korbs K oder ist kleiner als die Hälfte der Länge des Korbs K. Der Dentalpflegeeinsatz DPE belegt gemäß diesem Ausführungsbeispiel weniger als ein Viertel der Grundfläche des Korbs K.

Gemäß diesem Ausführungsbeispiel ist die Abschirmeinrichtung 200 ausgebildet, um das Abschirmmedium als eine senkrecht wirkende Luftbarriere zwischen dem ersten Reinigungsbereich und dem zweiten Reinigungsbereich zu erzeugen. Gemäß diesem Ausführungsbeispiel ist die Abschirmeinrichtung 200 ausgebildet, um die parallele Trennebene aus Wasser mit einer Skalierung auf einen kleineren Dentalpflegeeinsatz DPE als in Figur 4 dargestellt, zu erzeugen. Anders ausgedrückt bildet Figur 5 die in Figur 2 beschriebene Ausprägung mit Wasser in einer abweichenden Größenskalierung für einen kleineren zur Dentalinstrumentenaufbereitung und Pflege verwendeten Bereich ab.

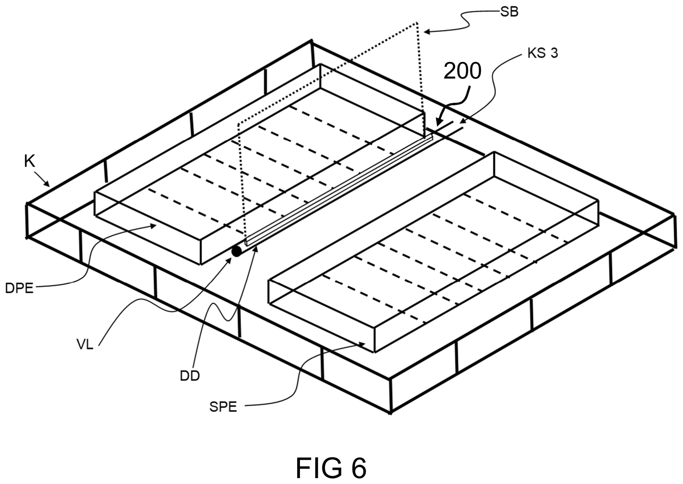

Figur 6 zeigt eine perspektivische Darstellung einer Abschirmeinrichtung 200 eines Reinigungsgeräts gemäß einem Ausführungsbeispiel. Dabei kann es sich um die in Figur 3 beschriebene Abschirmeinrichtung 200 handeln, mit dem Unterschied, dass die Düseneinrichtung gemäß diesem Ausführungsbeispiel lediglich eine der Düsen aufweist, die gemäß diesem Ausführungsbeispiel als eine Druckluftdüse DD ausgeformt ist, um das Abschirmmedium in Form von Druckluft in den Reinigungsraum auszugeben.

Die Abschirmeinrichtung 200 ist gemäß diesem Ausführungsbeispiel wie in Figur 4 beschrieben an dem Korb K und/oder dem Dentalpflegeeinsatz DPE angeordnet. Das Sprühbild SB der aus der Druckluftdüse DD ausgegebenen Druckluft ist gemäß diesem Ausführungsbeispiel rechteckig ausgeformt, um eine durchgehende Trennwand auszubilden. Eine Länge der Druckluftdüse DD und des Sprühbilds SB entspricht gemäß diesem Ausführungsbeispiel mehr als einer Hälfte einer Länge des Korbs K. Die Druckluftdüse DD ist gemäß einem Ausführungsbeispiel als eine Flachstrahldüse ausgeformt.

In Figur 6 ist in anderen Worten ausgedrückt ein beispielhafter Dentalpflegeeinsatz DPE in Form eines Spülkorbeinsatzes für Hand- und Winkelstücke und/oder Dentalturbinen mit Wasserabschirmung dargestellt. Die Abschirmeinrichtung 200 erzeugt gemäß diesem Ausführungsbeispiel eine parallele Trennebene aus Druckluft. Figur 6 zeigt eine in Figur 3 beschriebene Ausprägung mit Druckluft in ihrer Umsetzung im Korb K.

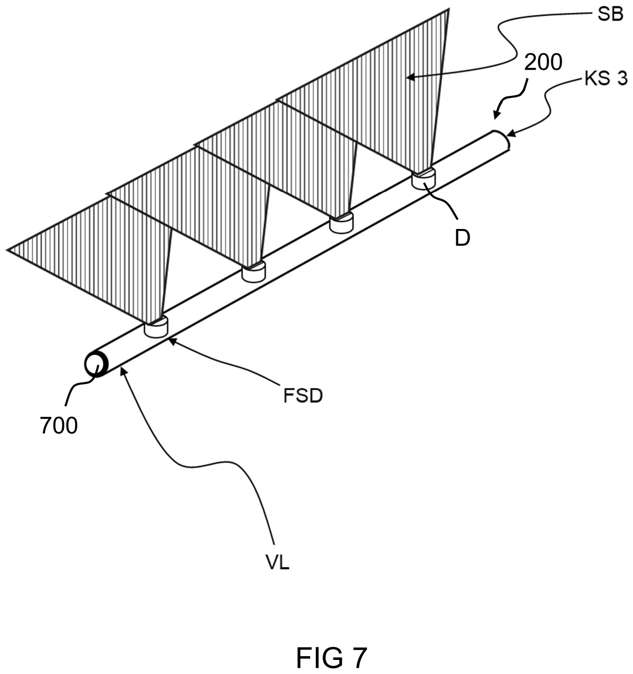

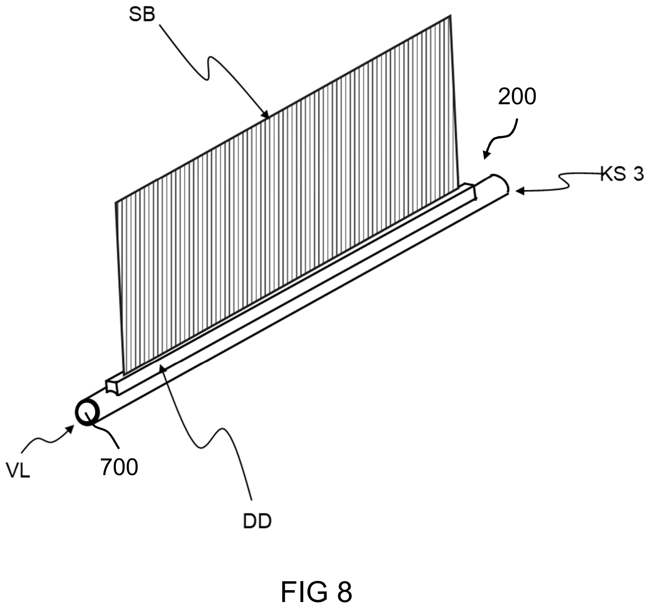

Figur 7 zeigt eine perspektivische Darstellung einer Abschirmeinrichtung 200 eines Reinigungsgeräts gemäß einem Ausführungsbeispiel. Dabei kann es sich um die in Figur 2, 4 oder 5 beschriebene Abschirmeinrichtung 200 handeln, mit dem Unterschied, dass die Düseneinrichtung gemäß diesem Ausführungsbeispiel vier der Düsen aufweist, die gleichmäßig zueinander beabstandet und/oder fluchtend zueinander entlang der Verteilleiste VL angeordnet sind. Ein der Koppelstelle KS3 gegenüberliegendes Ende 700 der Verteilleiste VL ist gemäß diesem Ausführungsbeispiel verschlossen, sodass über die Koppelstelle KS3 in die Verteilleiste VL zugeführtes Wasser lediglich über die vier Düsen D aus der Verteilleiste VL entweichen kann. Die Figuren 7 und 8 zeigen die Verteilleisten VL und die entstehenden Sprühbilder SB beider Ausprägungen mit Wasser oder Druckluft im Detail.

Figur 8 zeigt eine perspektivische Darstellung einer Abschirmeinrichtung 200 eines Reinigungsgeräts gemäß einem Ausführungsbeispiel. Dabei kann es sich um die in Figur 6 beschriebene Abschirmeinrichtung 200 handeln. Ein der Koppelstelle KS3 gegenüberliegendes Ende 700 der Verteilleiste VL ist gemäß diesem Ausführungsbeispiel verschlossen, sodass über die Koppelstelle KS3 in die Verteilleiste VL zugeführtes Wasser lediglich über die Druckluftdüse DD aus der Verteilleiste VL entweichen kann.

Figur 9 zeigt eine Frontalansicht einer Abschirmeinrichtung 200 eines Reinigungsgeräts gemäß einem Ausführungsbeispiel. Dabei kann es sich um die in Figur 6 oder 8 beschriebene Abschirmeinrichtung 200 handeln. Die Abschirmeinrichtung 200 ist gemäß diesem Ausführungsbeispiel wie in Figur 4 beschrieben an dem Korb K und/oder dem Dentalpflegeeinsatz DPE angeordnet. Beispielhaft ist eine Aufnahmeeinrichtung 206 dargestellt, an der zumindest ein medizinisches Instrument DI angebunden ist. Das zumindest eine medizinische Instrument DI, hier in Form eines Dentalinstruments, ist gemäß diesem Ausführungsbeispiel an einer Dentalinstrument-Adaption DIA des Dentalpflegeeinsatzes DPE oder einer Aufnahme der Verteilkammer angeordnet. Ein während des hier gezeigten Pflegebetriebs erzeugtes Pflegeöl-Sprühbild SP zeigt aus dem medizinischen Instrument DI austretendes und in den ersten Reinigungsbereich 400 eintretendes Pflegemittel. Das während eines Betriebs der Abschirmeinrichtung 200 mittels der Druckluftdüse DD erzeugte Sprühbild SB bildet gemäß diesem Ausführungsbeispiel eine Druckluftbarriere für das Pflegemittel, sodass kein Pflegemittel von dem ersten Reinigungsbereich 400 in den zweiten Reinigungsbereich 405 übertritt.

In Figur 9 ist eine Prinzipdarstellung einer Abschirmungsebene 900 aus Druckluft im Moment der Instrumentenpflege mit Pflegeöl während des Pflegebetriebs dargestellt. Gemäß einem alternativen Ausführungsbeispiel handelt es sich bei der Abschirmeinrichtung 200 um die in einer der Figuren 4, 5 oder 7 beschriebene Abschirmeinrichtung 200 zum Ausgeben des Abschirmmediums in Form von Wasser, sodass die Abschirmungsebene 900 gemäß dem alternativen Ausführungsbeispiel aus Wasser im Moment der Instrumentenpflege mit Pflegeöl während des Pflegebetriebs ausgeformt ist.

Figur 10 zeigt ein Ablaufdiagramm eines Ausführungsbeispiels eines Verfahrens 1000 zum Betreiben eines Reinigungsgeräts. Dabei kann es sich um das in einer der Figuren 2 bis 3 beschriebene Reinigungsgerät handeln, das die in einer der Figuren 2 bis 9 beschriebene Abschirmeinrichtung aufweist.

Das Verfahren 1000 weist einen Schritt 1005 des Durchführens und einen Schritt 1010 des Ausgebens auf. Im Schritt 1005 des Durchführens wird der Pflegebetrieb durchgeführt, um das in dem Reinigungsraum aufgenommene medizinische Instrument mit dem Pflegemittel zu pflegen. Im Schritt 1010 des Ausgebens wird das Abschirmmedium ausgegeben, um den ersten Reinigungsbereich des Reinigungsraums des Reinigungsgeräts von dem zweiten Reinigungsbereich des Reinigungsraums des Reinigungsgeräts zu separieren, um während des Pflegebetriebs ein Übertragen des Pflegemittels von dem ersten Reinigungsbereich in den zweiten Reinigungsbereich zu verhindern.

Das Verfahren 1000 weist gemäß diesem Ausführungsbeispiel ferner einen optionalen Schritt 1015 des Erkennens vor dem Schritt 1010 des Ausgebens auf, wobei im Schritt 1015 des Erkennens zumindest ein in dem zweiten Reinigungsbereich angeordnetes medizinisches Instrument erkannt wird, wobei der Schritt 1010 des Ausgebens ansprechend auf den Schritt 1015 des Erkennens durchgeführt wird.

## Patentansprüche

1. Reinigungsgerät (WD) für medizinische Instrumente (DI), wobei das Reinigungsgerät (WD) folgende Merkmale aufweist:
einen Reinigungsraum (SK) mit einem ersten Reinigungsbereich (400), in dem eine Aufnahmeeinrichtung (206) zum Aufnehmen zumindest eines der medizinischen Instrumente (DI) angeordnet oder anordenbar ist;
eine Pflegemittelleitung (105), die dazu ausgebildet ist, um ein Pflegemittel an eine Schnittstelle (KS2) zu der Aufnahmeeinrichtung (206) zu leiten, um das in oder an der Aufnahmeeinrichtung (206) aufgenommene medizinische Instrument (DI) während eines Pflegebetriebs mit dem Pflegemittel zu pflegen;
eine Abschirmeinrichtung (200), die innerhalb des Reinigungsraums (SK) angeordnet oder anordenbar ist, und die ausgebildet ist, um ein Abschirmmedium auszugeben, um den ersten Reinigungsbereich (400) von einem sich von dem ersten Reinigungsbereich (400) unterscheidenden zweiten Reinigungsbereich (405) des Reinigungsraums (SK) zu separieren, um während des Pflegebetriebs ein Übertragen des Pflegemittels von dem ersten Reinigungsbereich (400) in den zweiten Reinigungsbereich (405) zu verhindern; und
eine Abschirmmediumsleitung (205) welche dazu ausgebildet ist, um das Abschirmmedium an eine Koppelstelle (KS3) zu der Abschirmeinrichtung (200) zu leiten.

2. Reinigungsgerät (WD) gemäß Anspruch 1, bei der die Abschirmeinrichtung (200) eine Düseneinrichtung mit zumindest einer Düse (D) zum Ausgeben des Abschirmmediums in den Reinigungsraum (SK) aufweist.

3. Reinigungsgerät (WD) gemäß Anspruch 2, bei der die Düseneinrichtung eine Verteilleiste (VL) umfasst, die ausgebildet ist, um während des Ausgebens des Abschirmmediums in den Reinigungsraum (SK) das Abschirmmedium auf zumindest die Düse (D) oder eine Mehrzahl von Düsen (D) der Düseneinrichtung zu verteilen.

4. Reinigungsgerät (WD) gemäß einem der Ansprüche 2 bis 3, bei der die Düse (D) als eine Flachstrahldüse (FSD) ausgeformt ist, die ausgebildet ist, um das Abschirmmedium in einem flachen Strahl zur Erzeugung einer Trennwand zwischen dem ersten Reinigungsbereich (400) und dem zweiten Reinigungsbereich (405) auszugeben.

5. Reinigungsgerät (WD) gemäß einem der vorangegangenen Ansprüche, mit einem Korb (K) und/oder einem Instrumentenpflegeeinsatz (DPE) zur Aufnahme der medizinischen Instrumente (DI) in den Reinigungsraum (SK), wobei die Abschirmeinrichtung (200) in oder an dem Korb (K) und/oder Instrumentenpflegeeinsatz (DPE) angeordnet ist.

6. Reinigungsgerät (WD) gemäß einem der vorangegangenen Ansprüche, das die Abschirmmediumsleitung (205) in Form einer Wasserleitung und/oder ein Wassereinlaufventil (VEW) aufweist, wobei ein erstes Ende der Wasserleitung über die Koppelstelle (KS3) fluidisch mit der Abschirmeinrichtung (200) gekoppelt oder koppelbar ist und/oder ein zweites Ende der Wasserleitung fluidisch mit dem Wassereinlaufventil (VEW) zum Zuführen des Abschirmmediums in Form von Wasser in die Wasserleitung gekoppelt oder koppelbar ist.

7. Reinigungsgerät (WD) gemäß einem der vorangegangenen Ansprüche, das die Abschirmmediumsleitung (205) in Form einer Druckluftleitung aufweist, wobei ein erstes Ende der Druckluftleitung über die Koppelstelle (KS3) fluidisch mit der Abschirmeinrichtung (200) gekoppelt oder koppelbar ist und/oder ein zweites Ende der Druckluftleitung fluidisch mit einer Drucklufteinrichtung (b, DRV, DV, DVR, F, KS1, SV, 130) für das Reinigungsgerät (WD) gekoppelt oder koppelbar ist, um das Abschirmmedium in Form von Druckluft in die Druckluftleitung zu leiten.

8. Reinigungsgerät (WD) gemäß Anspruch 7, mit der Drucklufteinrichtung (b, DRV, DV, DVR, F, KS1, SV, 130) zum Bereitstellen der Druckluft.

9. Reinigungsgerät (WD) gemäß einem der Ansprüche 7 bis 8, bei dem das zweite Ende der Druckluftleitung zwischen einem Druckregelventil (DRV) der Drucklufteinrichtung (b, DRV, DV, DVR, F, KS1, SV, 130) und einem Druckluftventil (DV) der Drucklufteinrichtung (b, DRV, DV, DVR, F, KS1, SV, 130) fluidisch mit der Drucklufteinrichtung (b, DRV, DV, DVR, F, KS1, SV, 130) gekoppelt oder koppelbar ist.

10. Reinigungsgerät (WD) gemäß einem der Ansprüche 7 bis 9, bei dem die Druckluftleitung ein Abschirmventil (AV) zum Öffnen und/oder Schließen der Druckluftleitung aufweist.

11. Reinigungsgerät (WD) gemäß einem der vorangegangenen Ansprüche, mit einer Steuereinrichtung (210), die ausgebildet ist, um ein Aktivierungssignal auszugeben, das ausgebildet ist, um einen Betrieb der Abschirmeinrichtung (200) ansprechend auf den Pflegebetrieb zu aktivieren, um das Abschirmmedium vor und/oder während des Pflegebetriebs auszugeben.

12. Reinigungsgerät (WD) gemäß Anspruch 11, mit einer Erkenneinrichtung (215), die ausgebildet ist, um ein Erkennsignal bereitzustellen, das ein Erkennen eines in dem zweiten Reinigungsbereich (405) des Reinigungsraums (SK) angeordneten medizinischen Instruments (DI) repräsentiert, wobei die Steuereinrichtung (210) ausgebildet ist, um das Aktivierungssignal unter Verwendung des Erkennsignals auszugeben.

13. Reinigungsgerät (WD) gemäß einem der vorangegangenen Ansprüche, mit einer Pflegemittelbereitstellungseinheit (ÖL) zur Bereitstellung des Pflegemittels, wobei die Pflegemittelbereitstellungseinheit (ÖL) ausgebildet ist, um unter Verwendung eines Pflegemittelsignals das Pflegemittel über die Pflegemittelleitung (105) zu der in dem Reinigungsraum (SK) angeordneten Aufnahmeeinrichtung (206) zu fördern.

14. Verfahren (1000) zum Betreiben eines Reinigungsgeräts (WD) gemäß einem der vorangegangenen Ansprüche, wobei das Verfahren (1000) die folgenden Schritte aufweist:
Durchführen (1005) des Pflegebetriebs, um das in dem Reinigungsraum (SK) aufgenommene medizinische Instrument (DI) mit dem Pflegemittel zu pflegen; und
Ausgeben (1010) des Abschirmmediums, um den ersten Reinigungsbereich (400) des Reinigungsraums (SK) des Reinigungsgeräts (WD) von dem zweiten Reinigungsbereich (405) des Reinigungsraums (SK) des Reinigungsgeräts (WD) zu separieren, um während des Pflegebetriebs ein Übertragen des Pflegemittels von dem ersten Reinigungsbereich (400) in den zweiten Reinigungsbereich (405) zu verhindern.

15. Verfahren (1000) gemäß Anspruch 14, mit einem Schritt (1015) des Erkennens vor dem Schritt (1010) des Ausgebens, wobei im Schritt (1015) des Erkennens zumindest ein in dem zweiten Reinigungsbereich (405) angeordnetes medizinisches Instrument (DI) erkannt wird, wobei der Schritt des Ausgebens (1010) ansprechend auf den Schritt (1015) des Erkennens durchgeführt wird.
